(19) Europäisches Patentamt / European Patent Office / Office européen des brevets

(11) **EP 4 528 335 B1**

(12) **EUROPEAN PATENT SPECIFICATION**

(45) Date of publication and mention
of the grant of the patent:
**08.10.2025 Bulletin 2025/41**

(21) Application number: **24197226.4**

(22) Date of filing: **29.08.2024**

(51) International Patent Classification (IPC):
*G01T 1/24* *(2006.01)*    *G01T 1/29* *(2006.01)*

(52) Cooperative Patent Classification (CPC):
**G01T 1/247; G01T 1/2928**

(54) **SPECTRAL PHOTON COUNTING RADIATION DETECTOR STRUCTURES HAVING IMPROVED COUNT STABILITY AND METHODS OF OPERATING THEREOF**

SPEKTRALPHOTONZÄHLENDE STRAHLUNGSDETEKTORSTRUKTUREN MIT VERBESSERTER ZÄHLSTABILITÄT UND VERFAHREN ZUM BETRIEB DAVON

STRUCTURES DE DÉTECTEUR DE RAYONNEMENT À COMPTAGE DE PHOTONS SPECTRAL PRÉSENTANT UNE STABILITÉ DE COMPTAGE AMÉLIORÉE ET LEURS PROCÉDÉS DE FONCTIONNEMENT

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **22.09.2023 US 202363584734 P**

(43) Date of publication of application:
**26.03.2025 Bulletin 2025/13**

(73) Proprietor: **Redlen Technologies, Inc.
Saanichton, British Columbia V8M 0A5 (CA)**

(72) Inventors:
• **JACKSON, Michael K.
Saanichton, V8M 0A5 (CA)**
• **INIEWSKI, Krzysztof
Saanichton, V8M 0A5 (CA)**

(74) Representative: **Wichmann, Hendrik
Wuesthoff & Wuesthoff
Patentanwälte und Rechtsanwalt PartG mbB
Schweigerstraße 2
81541 München (DE)**

(56) References cited:
**US-A1- 2007 098 139    US-A1- 2016 203 620
US-A1- 2021 186 439    US-A1- 2021 382 188**

**Description**

FIELD

The present disclosure relates generally to radiation detectors, and more specifically to spectral photon counting radiation detector structures having improved count stability and methods of operating thereof.

BACKGROUND

Room temperature pixelated radiation detectors made of semiconductors, such as cadmium zinc telluride ($Cd_{1-x}Zn_xTe$ where $0<x<1$, or "CZT"), are gaining popularity for use in medical and non-medical imaging. These applications use the high energy resolution and sensitivity of the radiation detectors. US 2016/0203620 A1 discloses a computed tomography (CT) imaging apparatus which includes a radiation source configured to emit X-rays; a plurality of photon-counting detectors configured to detect X-rays emitted by the radiation source and generate a photon counting signal based on the detected X-rays; and processing circuitry to obtain a kV-waveform used by the radiation source to generate the X-rays during a scan of an object, and adjust at least one energy threshold dividing the photon counting signal into a plurality of spectra bins in accordance with the obtained kV-waveform. US 2007/0098139 A1 discloses a method and system of counting and tagging radiation energy received by a radiation detector, wherein the method and system are designed to dynamically control the sampling window or shaping time characteristics of a photon counting detector to accommodate variations of flux experienced by the detector so as to preserve optimum detector performance and prevent saturation during high flux conditions. US 2021/0382188 A1 discloses a radiation detector adapted for detecting leakage currents which comprises a direct conversion material for converting incident radiation, at least one first electrode and a plurality of second electrodes connected to surfaces of the direct conversion material for collecting each generated charges upon application of an electric field, at least one current measurement device, and a plurality of signal processing chains. US 2021/0186439 A1 discloses a method for generating an X-ray image dataset via an X-ray detector having a converter element and a multiplicity of pixel elements.

SUMMARY

According to the claimed invention, there is provided a detector structure which includes at least one radiation sensor including an array of pixel detectors, an application specific integrated circuit (ASIC) including a plurality of signal processing channel circuits electrically coupled to respective pixel detectors of the array of pixel detectors, each signal processing channel circuit configured to generate photon count data for multiple energy bins for a respective pixel detector of the array of pixel detectors, and at least one compensation circuit that receives photon count data for multiple energy bins from one or more signal processing channel circuits and adjusts a response characteristic of at least one signal processing channel circuit of the ASIC based on the received photon count data as further defined in the claims.

The at least one compensation circuit includes a threshold adjustment compensation circuit that is configured to adjust one or more threshold levels of the energy bins in at least one signal processing channel circuit of the ASIC based on the received photon count data as further defined in the claims.

In another embodiment, the ASIC further includes a current source coupled to an input node of at least one signal processing channel circuit, and the at least one compensation circuit includes a current adjustment compensation circuit that is configured to control the current source to selectively apply a compensation current to the input node of the at least one signal processing channel circuit based on the received photon count data

Detector arrays can be including a plurality of the above-described detector structures, where the radiation sensors of the plurality of detector structures form a continuous detector surface of the detector array.

Further embodiments include X-ray imaging systems including a radiation source configured to emit an X-ray beam, and a detector array including a plurality of the above-described detector structures that are configured to receive the X-ray beam from the radiation source through an intervening space configured to contain an object therein as further defined in the claims.

Useful for understanding the invention can be methods for training a feedback control algorithm for a spectral photon counting (SPC) detector having at least one radiation sensor coupled to an application specific integrated circuit (ASIC), the method including performing a training sequence that comprises exposing an SPC detector to a series of controllably varied X-ray flux levels, obtaining count stability metrics for the SPC detector on varying time scales, and providing the count stability metrics as inputs to an algorithm that adjusts one or more compensation parameters during a subsequent exposure to varying X-ray flux levels to drive the count stability behavior of the SPC detector to approach the count stability behavior of an ideal SPC detector.

Further embodiments according to the claimed invention include a method of using the claimed detector structure (a radiation detection method), comprising: providing radiation to at least one radiation sensor comprising an array of pixel detectors; generating photon count data for multiple energy bins for a respective pixel detector of the array of pixel detectors using an application specific integrated circuit (ASIC) including a plurality of signal processing channel circuits electrically coupled to respective pixel detectors of the array of pixel detectors; and adjusting a response characteristic of at least one signal processing channel circuit of the ASIC based on the photon count data for the multiple energy bins provided from the one or more signal processing channel circuits as further defined in the claims.

## BRIEF DESCRIPTION OF THE DRAWINGS

FIGS. 1A and 1B are functional block diagrams of an X-ray imaging system in accordance with various embodiments of the present disclosure.

FIG. 2A schematically illustrates a radiation detector including a pixelated radiation sensor coupled to a detector application specific integrated circuit (ASIC) according to various embodiments of the present disclosure.

FIG. 2B is a schematic block diagram illustrating a signal processing chain architecture of a detector ASIC for one channel of a spectral photon counting (SPC) radiation detector according to a comparative example of the present disclosure.

FIG. 2C is a schematic block diagram illustrating an alternative signal processing channel architecture of a detector ASIC for one channel of a spectral photon counting (SPC) radiation detector according to a comparative example the present disclosure.

FIG. 3 is a plot of current over time that illustrates the total current from photo-events in the presence of a baseline current.

FIG. 4A is a block diagram schematically illustrating a signal chain architecture for one channel of an SPC radiation detector including a threshold adjustment compensation circuit according to various embodiments of the present disclosure.

FIG. 4B is a block diagram schematically illustrating a signal chain architecture for one channel of an SPC radiation detector including a threshold adjustment compensation circuit according to another embodiment of the present disclosure.

FIG. 5 is a block diagram schematically illustrating a general arrangement for a threshold adjustment compensation circuit as shown in FIGS. 4A and 4B according to an embodiment of the present disclosure.

FIG. 6 is a block diagram schematically illustrating a signal chain architecture for one channel of an SPC radiation detector including a threshold adjustment compensation circuit and a temperature sensor according to various embodiments of the present disclosure.

FIG. 7A is a block diagram schematically illustrating a signal chain architecture for one channel of an SPC radiation detector including a current source and a current adjustment compensation circuit according to various embodiments of the present disclosure.

FIG. 7B is a block diagram schematically illustrating a signal chain architecture for one channel of an SPC radiation detector including a current source and a current adjustment compensation circuit according to according to another embodiment of the present disclosure.

FIG. 7C is a block diagram schematically illustrating a signal chain architecture for one channel of an SPC radiation detector including a current adjustment compensation circuit, a current source, and a temperature sensor according to various embodiments of the present disclosure.

FIG. 8 is a block diagram schematically illustrating an apparatus that may be used for training of a feedback control algorithm to be implemented in a compensation circuit for an SPC detector according to various embodiments of the present disclosure.

FIG. 9 is a block diagram schematically illustrating an imaging system that may be used for training of a feedback control algorithm to be implemented in a compensation circuit for an SPC detector according to various embodiments of the present disclosure.

FIG. 10 illustrates an X-ray imaging system including a phantom for providing stepwise flux modulation according to various embodiments of the present disclosure.

FIG. 11 illustrates an X-ray imaging system including a phantom having an array of absorbing rods according to various embodiments of the present disclosure.

## DETAILED DESCRIPTION

Embodiments of the present disclosure provide detector structures, such as radiation detector units and radiation detector modules, and detector arrays formed by assembling the detector structures, and methods of operating thereof, the various aspects of which are described herein with reference to the drawings.

The various embodiments will be described in detail with reference to the accompanying drawings. Wherever possible, the same reference numbers will be used throughout the drawings to refer to the same or like parts. References made to particular examples and implementations are for illustrative purposes, and are not intended to limit the scope of the invention or the claims. Any reference to claim elements in the singular, for example, using the articles "a," "an," or "the" is not to be construed as limiting the element to the singular. The terms "example," "exemplary," or any term of the like are used herein to mean serving as an example, instance, or illustration. Any implementation described herein as an "example" is not necessarily to be construed as preferred or advantageous over another implementation. The drawings are not drawn to scale. Multiple instances of an element may be duplicated where a single instance of the element is illustrated, unless absence of duplication of elements is expressly described or clearly indicated otherwise.

Ionizing radiation detectors, such as X-ray detectors, typically include a radiation-sensitive sensor material that is operatively coupled to detector read-out electronics. In most modern radiation detectors, the detector read-out electronics includes at least one semiconductor integrated circuit (IC), such as an application specific integrated circuit (ASIC) (which may also be referred to as a read out integrated circuit, or ROIC). Ionizing radiation detectors generally fall within two broad categories: energy integrating (EI) detectors and photon counting (PC) detectors. In EI detectors, the radiation-sensitive sensor material is commonly a solid-state scintillator material that is coupled to a photodiode. The scintillation light generated by the sensor material is proportional to both the energy of each photon incident on the sensor material as well as the number of incident photons per unit time. The photodiode converts the scintillation light to an electric signal that is amplified and integrated by the read-out electronics to produce the output signal.

In contrast, in a photon counting (PC) detector, the sensor material is typically a semiconductor material, such as cadmium telluride (CdTe), cadmium zinc telluride (CZT), silicon (Si), gallium arsenide (GaAS), etc., that is configured to directly detect photon interactions occurring within the sensor material. Photon interactions within the sensor material produce a cloud of charge carriers (e.g., via the photoelectric effect). A bias voltage applied to the sensor material produces an electric field that causes the charge carriers produced by the photon interactions to be swept towards electrodes located on opposite sides of the sensor material. The charge that is received at the electrodes produces a signal that may be amplified and converted into a voltage signal by the detector read-out electronics. The read-out electronics may additionally include a "comparator" that compares the amplitude of the voltage signal to a pre-set threshold value, and a "counter" (which may also be referred to as an "accumulator") that measures the total number of voltage signals that exceed the pre-set threshold value, thus providing a total count of photons that impinge on the detector. The pre-set threshold is typically set to distinguish between "true" photon interaction event signals and electronic noise signals. Thus, a PC detector may have less noise than an energy integrating (EI) detector.

A spectral photon counting (SPC) detector is a type of PC detector that utilizes multiple pre-set threshold values to sort each of the detected voltage signals into different "bins" representing different energies of the incident photons. The read-out electronics of an SPC detector may sort incident photons into one or more energy bins by comparing the amplitudes of each of the detected voltage signals to a plurality of different threshold values. The total number of energy bins may be between 2 and 12 bins, for example. Thus, an SPC detector may provide both image information and measurements of the energy of the detected photons. An SPC detector may also be referred to as an energy-discriminating radiation detector.

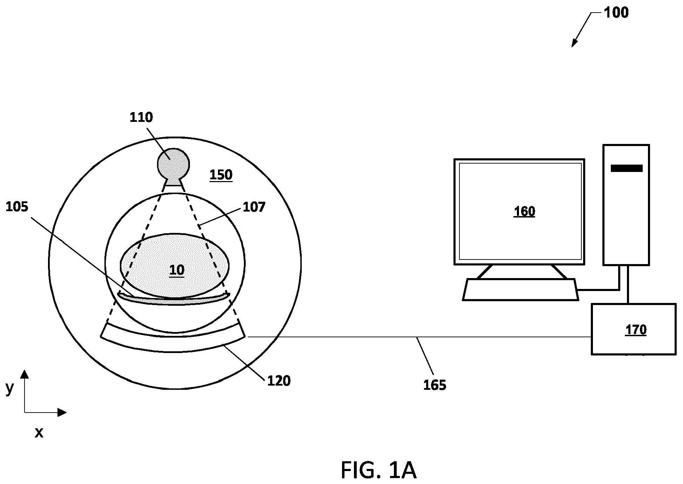

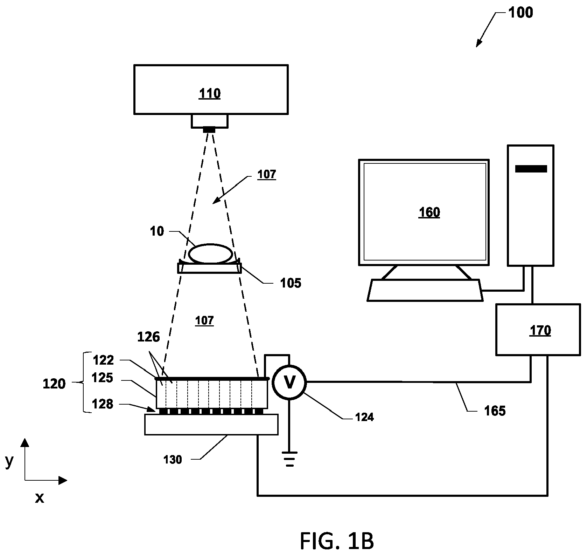

SPC detectors have been used for X-ray imaging applications, including for X-ray computed tomography (CT) imaging. FIGS. 1A and 1B are functional block diagrams of an X-ray imaging system 100 in accordance with various embodiments. The X-ray imaging system 100 may include an X-ray source 110 (i.e., a source of ionizing radiation), and a spectral photon counting radiation detector 120. The X-ray imaging system 100 may additionally include a patient support structure 105, such as a table or frame, which may rest on the floor and may support an object 10 to be scanned. In some embodiments, the object 10 may be a biologic subject (i.e., a human or animal patient). The support structure 105 may be stationary (i.e., non-moving) or may be configured to move relative to other elements of the X-ray imaging system 100, such as the X-ray source 110.

As shown in FIG. 1A, the X-ray source 110 may be mounted to a gantry 150 and may move or remain stationary relative to the object 10. The X-ray source 110 is configured to deliver ionizing radiation to the radiation detector 120 by emitting an X-ray beam 107 toward the object 10 and the radiation detector 120. After the X-ray beam 107 is attenuated by the object 10, the beam of radiation 107 is received by the radiation detector 120.

The radiation detector 120 may be segmented or configured into a large number of small "pixel" detectors, as described in further detail below. The radiation detector 120 may a spectral photon counting (SPC) detector that includes a radiation-sensitive sensor material, such as semiconductor material, coupled to detector read-out electronics (e.g., one or more above-described ASICs).

A control unit 170 may be configured to control the operations of the X-ray source 110 and the radiation detector 120. The control unit 170 may be coupled to and operated from a computing device 160. Alternatively, the computing device 160 and the control unit 170 may be integrated together as one device.

In the exemplary embodiment shown in FIGS. 1A and 1B, the X-ray imaging system 100 is an X-ray computed tomography (CT) imaging system 100. The CT imaging system 100 may include a gantry 150, which may include a moving part, such as a circular, rotating frame with the X-ray source 110 mounted on one side and the radiation detector 120 mounted on the other side. The radiation detector 120 may have a curved shape along its long axis (i.e., the x-axis direction in FIG. 1A) such that each of the pixel detectors along the length of the radiation detector may face towards the focal spot of the X-ray source 110. The gantry 150 may also include a stationary (i.e., non-moving) part (not shown in FIG. 1A), such as a support, legs, mounting frame, etc., which rests on the floor and supports the moving part. The X-ray source 110 may emit a fan-shaped or cone-shaped X-ray beam 107 as the X-ray source 110 and the radiation detector 120 rotate on the moving part of the gantry around the object 10 to be scanned. After the X-ray beam 107 is attenuated by the object 10, the X-ray beam 107 is received by the radiation detector 120. The curved shape of the radiation detector 120 may allow the CT imaging system 100 to most effectively reject radiation scattered by the object 10.

For each complete rotation of the X-ray source 110 and the radiation detector 120 around the object 10, one cross-sectional slice of the object 10 may be acquired. As the X-ray source 110 and the radiation detector 120 continue to rotate, the radiation detector 120 may take numerous snapshots called "views". Typically, about 1,000 profiles are taken in one rotation of the X-ray source 110 and the radiation detector 120. The X-ray source 110 and the detector 120 may slowly move relative to the patient along a horizontal direction (i.e., into and out of the page in FIG. 1A) so that the detector 120 may capture incremental cross-sectional profiles over a region of interest (ROI) of the object 10, which may include the entire object 10. The data acquired by the radiation detector 120 may be passed along to the computing device 160 that may be located remotely from the radiation detector 120 via a connection 165. The connection 165 may be any type of wired or wireless connection. If the connection 165 is a wired connection, the connection 165 may include a slip ring electrical connection between any structure (e.g., gantry) supporting the radiation detector 120 and a stationary support part of the support structure, which supports any part (e.g., a rotating ring). If the connection 165 is a wireless connection, the radiation detector 120 may contain any suitable wireless transceiver to communicate data with another wireless transceiver that is in communication with the computing device 160. The computing device 160 may include processing and imaging applications that analyze each profile obtained by the radiation detector 120, and a full set of profiles may be compiled to form a three-dimensional computed tomographic (CT) reconstruction of the object 10 and/or two-dimensional images of cross-sectional slices of the object 10.

Various alternatives to the design of the X-ray imaging system 100 of FIGS. 1A and 1B may be employed to practice embodiments of the present disclosure. X-ray imaging systems may be designed in various architectures and configurations. For example, an X-ray imaging system may have a helical architecture. In a helical X-ray imaging scanner, the X-ray source 110 and radiation detector 120 are attached to a freely rotating gantry 150. During a scan, a table moves the object 10 smoothly through the scanner, or alternatively, the X-ray source 110 and detector 120 may move along the length of the object 10, creating helical path traced out by the X-ray beam. Slip rings may be used to transfer power and/or data on and off the rotating gantry 150. In other embodiments, the X-ray imaging system may be a tomosynthesis X-ray imaging system. In a tomosynthesis X-ray scanner, the gantry may move in a limited rotation angle (e.g., between 15 degrees and 60 degrees) in order to detect a cross-sectional slice of the object 10. The tomosynthesis X-ray scanner may be able to acquire slices at different depths and with different thicknesses that may be reconstructed via image processing.

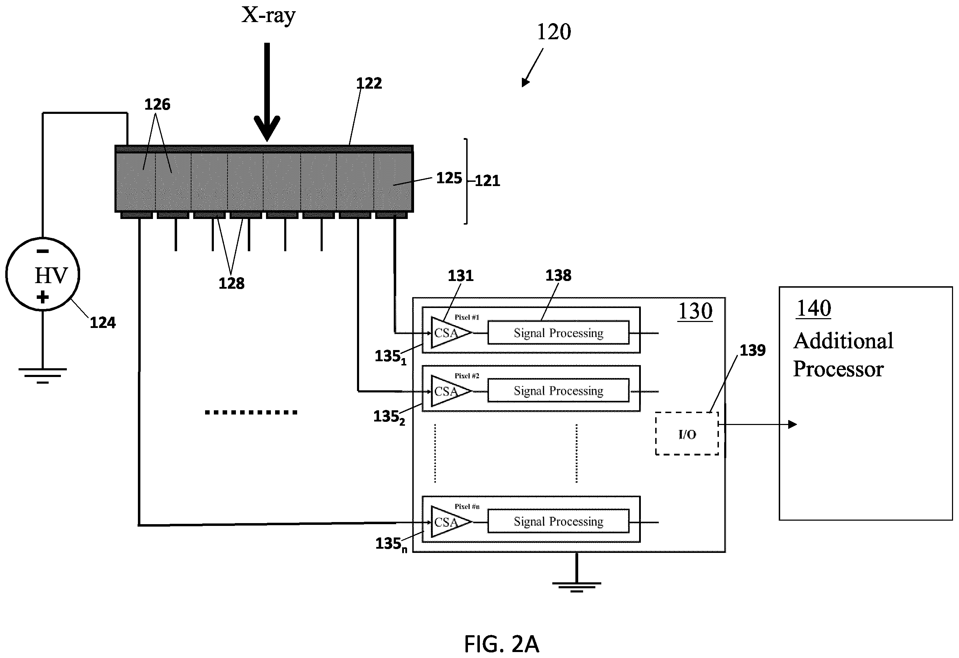

FIG. 2A schematically illustrates a radiation detector 120 including a pixelated radiation sensor 121 coupled to a detector application specific integrated circuit (ASIC) 130 according to various embodiments of the present disclosure. The radiation sensor 121 may be controlled by a high voltage bias power supply 124 that may selectively create an electric field between an anode 128 and cathode 122 pair coupled thereto. In one embodiment, the radiation sensor 121 includes a plurality of anodes 128 and one common cathode 122 electrically connected to the power supply 124. Each of the anodes 128 may define a different pixel detector 126 of a pixelated radiation detector 120. The radiation sensor 121 may include a detector material 125, such as a semiconductor material disposed between the anode(s) 128 and cathode 122 and thus configured to be exposed to the electrical field therebetween. In various embodiments, the radiation sensor 121 may be arranged such that the surface of the detector material 125 over which the cathode 122 is located faces towards the X-ray source 110 (see FIG. 1B). Thus, X-ray photons from the X-ray source 110 may impinge on the cathode-side of the radiation sensor 121. The semiconductor material 125 may include any suitable semiconductor material for detecting X-ray radiation disposed between the anode(s) 128 and cathode 122 and thus configured to be exposed to the electrical field therebetween. In various embodiments, the semiconductor material of the radiation sensor 121 may include a II-VI semiconductor material, such as cadmium telluride, cadmium zinc telluride (i.e., CdZnTe or "CZT"), cadmium selenide telluride, and cadmium zinc selenide telluride. Other suitable semiconductor materials are within the contemplated scope of disclosure.

An above-described detector application specific integrated circuit (ASIC) 130 may be coupled to the anode(s) 128 of the radiation sensor 121. The detector ASIC 130 may receive signals (e.g., charge or current) from the anode 128(s) and be configured to provide data to and be controlled by a control unit 170 (see FIG. 1B). The signals received by the detector ASIC 130 may be in response to photon interaction events occurring within the radiation-sensitive semiconductor material of the detector material 125. Accordingly, the signals received by the detector ASIC 130 may be referred to as "event detection signals."

The detector ASIC 130 may include a semiconductor integrated circuit (IC) die that includes a substrate (e.g., a silicon substrate) including a semiconductor material layer over a surface of the substrate and a plurality of circuit elements (e.g., transistors, resistors, capacitors, inductors, diodes, etc.) formed on and/or in the semiconductor material layer. The circuit elements of the detector ASIC 130 may be configured to perform signal processing operations on event detection signals received from the anode(s) 128 of the radiation sensor 121. In particular, each anode 128 of the radiation sensor 121 may be electrically coupled to an input node of a respective signal processing chain or "channel" $135_1$, $135_2$, ...$135_n$, of the detector ASIC 130. Thus, in various embodiments, event detection signals from each anode 128 of the radiation sensor 121 may be processed by a separate channel $135_1$, $135_2$, ... $135_n$ of the ASIC 130. Accordingly, the total number of signal processing channels $135_1$, $135_2$, ... $135_n$ of the ASIC 130 may be at least as great as the total number of pixel detectors 126 of the radiation sensor(s) 121 that are coupled to the ASIC 130. Each of the channels $135_1$, $135_2$, ... $135_n$ of the ASIC 130 may include a respective amplifier 131 and signal processing circuitry 138.

An ASIC 130 for a spectral photon counting (SPC) radiation detector 120 may include a plurality (e.g., hundreds) of identical or substantially-identical signal processing channels 135 as shown in FIG. 2A. Each channel 135 may be electrically coupled to an anode electrode 128 of a particular pixel detector 126 and may output photon count data of photon interaction events occurring within the particular pixel detector 126 over multiple energy bins. Referring again to FIG. 2A, the ASIC 130 may further include input/output (I/O) circuitry 139 that may be configured to transmit the photon count data from the ASIC 130 to another electronic component of the imaging system 100. In some embodiments, the I/O circuitry 139 may include high-speed I/O circuitry, such as low voltage differential signaling (LVDS) transmission circuitry.

Referring once again to FIG. 2A, in addition to the above-described ASIC 130, the read-out circuitry for the radiation detector 120 may include at least one additional processor 140, such as a field programmable gate array (FPGA). Other suitable processors are within the contemplated scope of disclosure. The at least one additional processor 140 may function as a downstream aggregator of photon count data output from the ASIC 130 and may optionally perform additional signal processing on the photon count data. In some embodiments, the at least one additional processor 140 may be used for configuration of the ASIC 130.

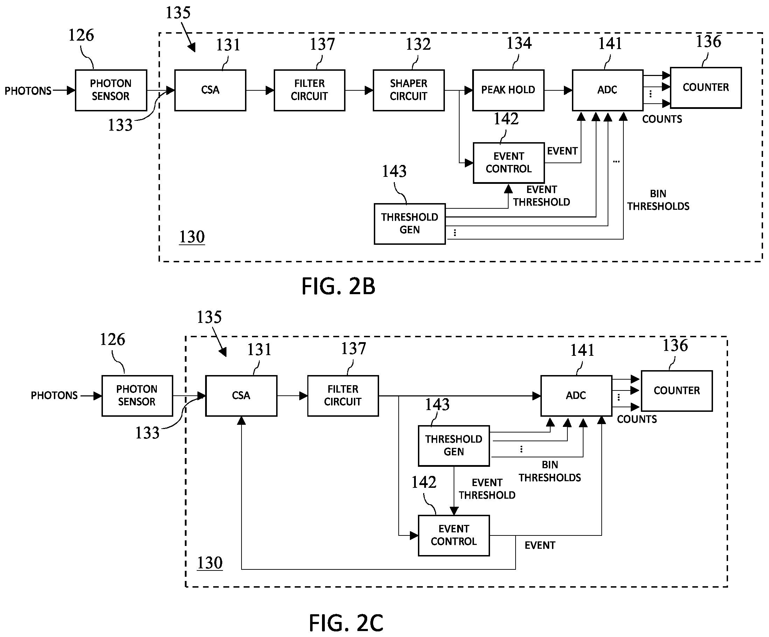

FIG. 2B is a block diagram schematically illustrating an analog signal chain architecture for one channel of a spectral photon counting (SPC) radiation detector according to a comparative example. Referring to FIG. 2B, the photon sensor (e.g., an above-described pixel detector 126) converts an incident X-ray photon to an electrical charge proportional to the photon energy. Detection signals (e.g., analog charge signals) from the photon sensor may be received at an input node 133 for the signal processing channel 135, which may be, for example, a bond pad located on a surface of the ASIC 130. The detection signals may be fed to an amplifier 131 that may be configured to convert the charge signal to a voltage signal. In some embodiments, the amplifier 131 may be a charge sensitive amplifier (CSA). The CSA may integrate the current to provide a voltage signal proportional to the photogenerated electron charge. The CSA may include a resistor in parallel with a feedback capacitor to limit the DC response. A filter circuit 137 may be included for pole-zero compensation of the CSA response. Alternatively, other types of amplifiers 131, such as a trans-impedance amplifier (TIA), may be utilized.

The signal processing channel 135 may additionally include a shaper circuit 132 that may be configured to "shape" the signal that is output by the amplifier 131. The shaper circuit 132 may convert the step-like filter output to a peaked form. The shaper circuit 132 may include a combination of first-order high-pass and low-pass filters, which may be referred to as an RCCR shaper. The output of the shaper circuit 132 may be processed by a peak hold circuit 134 to allow sampling by the analog to digital converter (ADC) 141.

The output of the shaper circuit 132 may also be provided to an event control block 142 that may be configured to generate a signal (i.e., an EVENT signal) when the output from the shaper circuit 132 exceeds an event threshold. When the EVENT signal is triggered, the ADC 141 may convert the signal to a digital format.

The threshold generator 143 may provide a plurality of threshold levels, including a first threshold level (also known as the trigger threshold level) used to indicate that a photon detection event has occurred, and one or more additional threshold levels (typically between 2 and 8 threshold levels), used to sort the detection signal into the relevant energy bin(s). For example, to detect photons having an energy over 20 keV, the first threshold level may be set at a threshold voltage, $V_{TH0}$, that is the equivalent of the signal produced by a 20 keV photon interaction event. The additional threshold levels may be set at threshold voltages, $V_{TH1}$, $V_{TH2}$, ... $V_{THn}$ used to define different ranges or "bins" of photon energies. The threshold voltages, $V_{TH0}$, $V_{TH1}$, $V_{TH2}$, ...$V_{THn}$, may be determined using a calibration process. Thus, in an example where $V_{TH0}$ corresponds to a cut-off reference photon energy of 20 keV, $V_{TH1}$ corresponds to a cut-off reference photon energy of 50 keV, and $V_{TH2}$ corresponds to a cut-off reference photon energy of 80 keV, photons having energies between 20-50 keV may be classified in a first energy bin, photons having energies between 50-80 keV may be classified in a second energy bin, and photons having energies > 80 keV may be classified in a third energy bin.

The ADC 141 may convert the signal from the shaper circuit 132 to a digital format by comparing the signal received from the shaper circuit 132 to the series of threshold levels, and incrementing the count of detected photon interaction events within the relevant energy bin(s) of the counter/accumulator 136, which may sum the counts within each energy bin over time.

FIG. 2C is a block diagram schematically illustrating an alternative signal chain architecture for one channel of a spectral photon counting (SPC) radiation detector according to another comparative example. The architecture shown in FIG. 2C may be referred to as a switched-analog design. In this design, a shaper circuit 132 and/or a peak hold circuit 134 as described above with reference to FIG. 2B may not be included. The signal from the amplifier 131 may be provided to a filter circuit 144, that may include a high-pass filter. The amplifier 131 (e.g., CSA) may be a switched integrator with only a capacitor in the feedback loop. This capacitor may be reset after an event occurs by closing a switch in parallel with the capacitor. When the event control block 142 event control generates an EVENT signal to indicate an event has occurred, the ADC 141 may sample the signal amplitude at a controlled time after the EVENT signal, allowing the filter circuit to reach its full amplitude. The ADC 141 may compare the signal amplitude to the plurality of threshold levels provided by the threshold generator 143, and the ADC 141 output may be routed to the counter/accumulator 136, which may sum the counts within each energy bin over time.

In some embodiments, a radiation detector 120 may have a modular configuration including a plurality of detector modules mounted on a common support structure, such as a detector array frame, to form a detector array (also known as a detector module system (DMS)) that includes a plurality of pixel detectors 126 extending over a continuous one-dimensional (1D) or two-dimensional (2D) detector array surface. Each detector module may include at least one above-described radiation sensor 121, at least one ASIC 130 electrically coupled to the at least one radiation sensor, and a module circuit board. The module circuit board may support transmission of electrical power, control signals, and data signals between the module circuit board and the at least one ASIC 130 and the at least one radiation sensor 121 of the detector module, and may further support transmission of electrical power, control signals, and data signals between the module circuit board and the control unit 170 of the X-ray imaging system 100, other module circuit boards of the detector array, and/or a power supply for the detector array.

An ideal spectral photon counting (SPC) radiation detector, such as a PCCT detector, would exhibit counting performance that is independent of the history of exposure of the detector, i.e., it will have count values that depend solely upon the instantaneous spectrum and intensity of the radiation incident upon the pixel detector. In practice, direct conversion PCCT detectors exhibit variability in the counting response either per bin or in aggregate (e.g., an open bin) due to the history of exposure of the detector. For example, CZT detector response is known to vary with exposure for several reasons including the trapping of photocarriers in the bulk CZT, trapping of photocarriers in the regions (i.e., "streets") between adjacent anode electrodes, and accumulation of photoelectrons at the anode interface. In addition, the change of digital activity within the ASIC may result in a change in heat generation and an accompanying, time-varying rise in ASIC temperature, which may affect counting thresholds. These imperfections rarely result in the complete loss of an event, that is, an X-ray photon absorption being undetected. Rather, the impact is a slight shift of the estimated energy of the event, which can be described as a "spectral shift." The amount of spectral shift needs to be kept very small to maintain good image quality, e.g., to achieve 0.1% counting stability in typical conditions may require shifts as low as 0.09eV or about 20e (i.e., about 20 electrons).

The effect of the time-varying counting response to fixed X-ray input may be described as the stability of the detector, meaning the stability of the photon counts when the input flux is constant. The stability is usually worst for large changes in flux. The stability of a radiation detector may be characterized by applying a step flux response and quantifying the count stability using a suitable metric. For example, the stability may be quantified by the following:

$$s_t = \frac{B-A}{A} \quad (\text{Eq. 1})$$

where $s_t$ is the stability over a given timeframe $t$, $B$ is the average count rate during some interval during time t, and $A$ is the average rate in some time just after the flux change has occurred. We may refer, for example to "1 second stability," where A is the average count rate from 0 to 50ms, and B is the average count rate from 700 to 1000 ms, where 0 ms is defined as the time at which the flux change has been completed.

It may be possible to minimize the spectral shift and improve count rate stability through the design of the radiation sensor 121. For example, U.S. Patent Application No. 18/318,987, filed on May 23, 2023, describes various techniques for minimizing spectral shift and improving count rate stability reducing or eliminating the bulk electric field change and/or the change due to leakage current in an ionizing radiation detector. However, such solutions may incur costs or require process control that is challenging. Other solutions, such as improved temperature control, may require complex solutions or extra power dissipation in the detector.

The small energy changes in the estimated energy of an event compared to the estimated energy of the same event at another time may be described as a "spectral shift" if the energy of all events is shifted equally, independent of energy. Such changes may additionally be described as a "spectral distortion" if there is a variation of the spectral shift with photon energy.

The spectral shift can be either negative (detected energy decreases, causing open-bin count to decrease) or positive (detected energy increases, open-bin counts increase). As discussed above, these shifts may be due to a change in the signal from the CZT, once integrated by the charge-sensitive amplifier (CSA) used in the front-end of typical PCCT ASICs. Alternatively, a temperature-induced shift of the threshold levels used in digitizing the ASIC outputs can have a similar effect. In practical detectors, both effects may be occurring at the same time. One important function in PCCT detectors is the thresholding of the estimated charge, i.e., the assignment of a given count to a specific bin, where the number of bins are from three to eight in typical systems. Because the charge developed in direct detection radiation sensors like those made from CZT is directly proportional to the energy of the X-ray photon that initiated the event, this allows determination of the spectrum of the detected photons, which enables many advantages of PCCT systems compared to older energy integrating detector (EID) systems.

The bin thresholds in SPC detectors may be adjusted for each pixel using a variety of calibration schemes, but in general, the thresholds remain constant during normal operation. Unfortunately, it is not generally possible to correct spectral shifts after the fact, i.e., in post processing of the count data, because binning of photons into wide energy bins involves loss of information. For this reason, it is impossible to correct this loss of information in post processing unless the spectral shape of the radiation incident upon the detector is known, which is generally not the case. The inability to correct with post-processing is worst in applications involving diverse spectral shapes, such as in imaging applications using contrast agents, which is of primary interest in PCCT. This problem occurs in both open-bin and closed-bin counting.

In prior art PCCT circuitry, implemented in application-specific integrated circuits (ASICs), spectral shifts may occur for one or more of the following reasons:

1. Bulk photocarrier trapping in the sensor material causes electric field changes that alter the event charge estimated by the ASIC.

2. Photoelectrons becoming trapped in the "streets" between adjacent anodes causing counting changes.

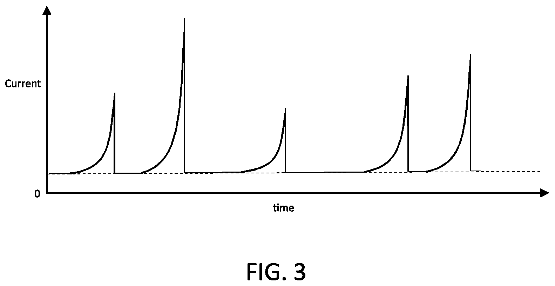

3. It has been demonstrated that if current from photo-events, which consists of short transient pulses, is accompanied by an additional steady DC current flow, the ASIC response to the pulses is generally increased. This spectral shift to higher energies is known as baseline shift. FIG. 3 is a plot of current over time that illustrates the total current from photo-events (transient pulses) in the presence of a baseline current, where the baseline current level is indicated by the dashed line. It is generally thought that such baseline current is substantially constant over long periods of time. In that case the baseline current is generally described as dark current or leakage current, meaning that the baseline current flow in the presence of X-ray pulses is substantially the same as the baseline current flow in the dark. However, it has been recognized that certain radiation sensors, due to the nature of the anode and/or cathode contacts, may exhibit a substantial variation in the baseline current level. This variation may occur, for example, due to the accumulation of photoelectrons at the interface between the sensor material (e.g., CZT) and the anode metal.

4. In addition, there can be effects in the ASIC itself that have nothing to do with the sensor, but are due to internal design choices in the ASIC circuitry. For example, it has been demonstrated that DC current consumption in the digital portions of an ASIC will increase with increasing circuit activity, which may correspond to increasing photo-event rate, i.e., with increasing flux. This may cause a change in power dissipation of the ASIC and a corresponding temperature shift, which may cause threshold shifts. Another example is that a change in DC current may affect power supply voltages due to resistive (I-R) drops, and thereby cause shifts in output levels from the threshold generation circuits. There are many different potential circuit effects that can lead to threshold shift with flux.

Various embodiments include a spectral photon counting (SPC) radiation detector 120 that compensates for the effects of spectral shift using the detector electronics (e.g., the ASIC 130).

The various embodiments of the present disclosure are based, at least in part, on the recognition that the threshold shift behavior of a detector is relatively consistent. While there may be some noise, or some small variations in response to the same stimuli, most of the imperfections in spectral photon counting detectors are consistent. For example, the change in detector counting response to a known flux exposure is quite consistent. In an operational phase while scanning a patient, the "correct" counting result that would be obtained by an ideal detector is not generally known. However, the Applicants have recognized that in a training phase without a patient present, i.e., in a laboratory or production environment, known stimuli may be applied in which the "correct" counting results expected from an ideal detector may be known *a priori.* Furthermore, many training runs may be repeated without any deleterious effects to patients due to high X-ray exposure.

Accordingly, the actual detector response may be characterized during a training phase, where, due to the relative consistency in threshold shift behavior of SPC detectors, it may be expected that the detector response will be substantially the same in an operational phase when exposed to the same stimuli. The training phase may be used to determine how closely the actual detector response is to the "correct" response of an ideal detector.

Various embodiments may include one or more compensation circuits within the signal processing chain of the detector electronics (e.g., the ASIC 130). The one or more compensation circuits may be configured to compensate for spectral shift by adjusting the response of the detector to more closely approximate the expected response of an "ideal" detector. The adjustments provided by the one or more compensation circuits may be based on a characterization of detector behavior during a prior training procedure. The compensation circuit includes a threshold adjustment circuit that is configured to adjust one or more threshold levels of the energy bin(s) as further defined in the claims (of the SPC detector). The adjustment of one or more threshold levels may be made dynamically in real-time (e.g., during an imaging operation) and may be based on count data provided to the threshold adjustment circuit. Alternatively, or in addition, a compensation circuit may include a current source adjustment circuit that may be configured to selectively apply a compensation current to the input node of one or more signal processing channels 135 of the ASIC 130. The compensation current may compensate for variations in the baseline or "leakage" current within the radiation sensor.

In some embodiments, the compensation circuit may utilize the output count signals from a given pixel, and in some cases from other pixels, to generate a control signal (which may also be referred to as a "count signal"). The count signal may be related to the photon flux incident on the given pixel(s). Accordingly, the compensation circuit may respond to changes in the count signal by adjusting a detector response characteristic, such as by adjusting one or more energy thresholds and/or by providing a compensation current to one or more signal processing channels 135. In some embodiments, the compensation circuit may include a plurality of filters and a weighted summing network that may be used to process the photon count data and generate the count signal. In some embodiments, the count signal may be proportional to photocarrier generation within the sensor material. In some embodiments, the count signal may be proportional to the rate of detection events processed by the ASIC.

In some embodiments, the compensation circuit configuration may be determined using data acquired during training runs, using as a desirability score the degree of imperfection of the detector response. Such a process may proceed by established machine learning techniques.

Various embodiments may be used to improve the response of a given radiation detector, so that it will behave in a more ideal manner. In this way a non-ideal detector may be made more ideal. Accordingly, detector performance, including count stability of the detector during changing flux conditions, may be improved.

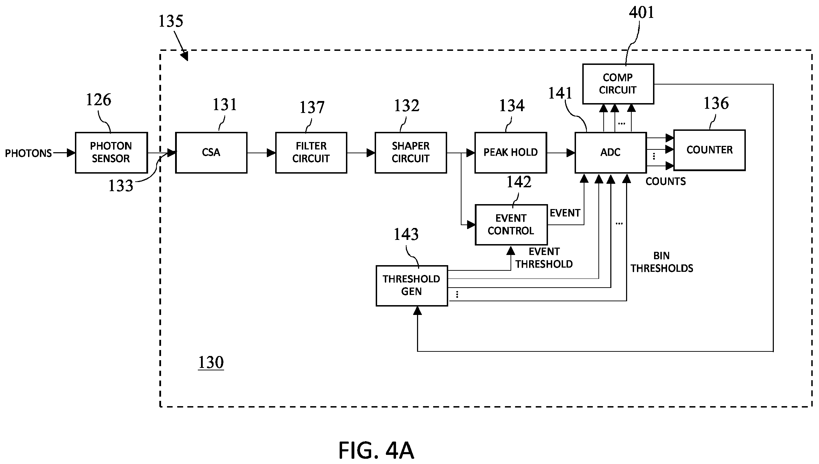

FIG. 4A is a block diagram schematically illustrating a signal chain architecture for one channel 135 of a spectral photon counting (SPC) radiation detector including a threshold adjustment compensation circuit 401 according to various embodiments of the present disclosure. The signal chain architecture shown in FIG. 4A may be derived from the analog signal chain architecture described above with reference to FIG. 2B. Thus, repeated discussion of like features is omitted for brevity. Referring to FIG. 4A, the threshold adjustment compensation circuit 401 may receive photon count data from the ADC 141 and/or from the counter/accumulator 136. Based on the photon count data, the threshold adjustment compensation circuit 401 may generate signals that are used to adjust the event thresholds set by the threshold generator 143 in the signal processing channel 135 for the pixel. In some embodiments, the adjustments may be made on a threshold-to-threshold basis, meaning that each threshold defining the different ranges or "bins" of photon energies may be adjusted individually. In other embodiments, a single adjustment may be applied to all the bin thresholds within the signal processing channel 135 for the pixel.

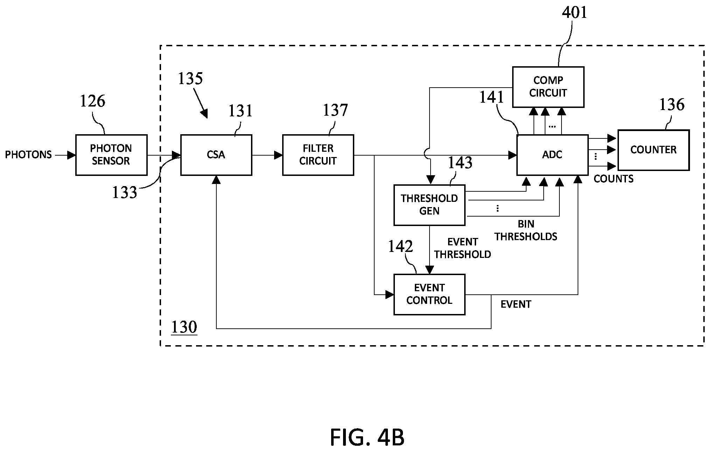

FIG. 4B is a block diagram schematically illustrating a signal chain architecture for one channel of a spectral photon counting (SPC) radiation detector including a threshold adjustment compensation circuit 401 according to another embodiment of the present disclosure. The signal chain architecture shown in FIG. 4B may have a switched-analog design as described above with reference to FIG. 2C. As in the embodiment of FIG. 4A, the threshold adjustment compensation circuit 401 may receive photon count data from the ADC 141 and/or from the counter/accumulator 136, and the threshold adjustment compensation circuit 401 may generate a signal that is used to adjust the event thresholds set by the threshold generator 143 in the signal processing channel 135 for the pixel.

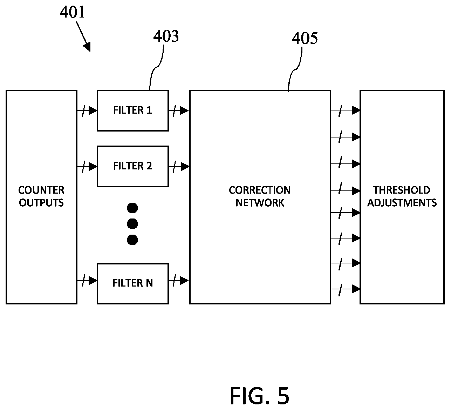

FIG. 5 is a block diagram schematically illustrating a general arrangement for a threshold adjustment compensation circuit 401 as shown in FIGS. 4A and 4B. The threshold adjustment compensation circuit 401 may include one or more digital filters 403 and a correction network 405. The threshold adjustment compensation circuit 401 may receive photon count data from a single pixel, a group of pixels (e.g., a group of neighboring pixels), or the entire set of pixels within a given detector structure, which may include at least one above-described radiation sensor 121 and at least one ASIC 130 electrically coupled to the at least one radiation sensor 121. The count data may be filtered using a plurality of digital filters 403 as illustrated in FIG. 5. The filters 403 may be time-scale low pass filters. In some embodiments, the filter constants and/or the time constants of the filters 403 may be set to correspond to the physical time constants for the changes in electric field that occur in the sensor material (e.g., CZT) in response to changes in the incident x-ray flux. In some embodiments, the filter functions and/or filter time constants may be set based on a training procedure described in further detail below.

The outputs from the filters 403 may be provided to the correction network 405. In some embodiments, the output from the filters 403 may include filtered count data for each of the energy bins from one or more pixels. The correction network 405 may include a weighted summing network that may use the outputs from the filters to generate a count signal that may be used to adjust the event thresholds for one or more energy bins in one or more pixels of the detector. In cases where the physical behavior being corrected is related to photocarrier density, in some embodiments the correction network 405 may generate the count signal by summing the filtered count data using an equation of the form:

$$C_i = \sum_{j=1}^{M} \alpha_j C_{ij} \, C_i$$

where $C_{ij}$ are the count values for bin $j$ for pixel $i$, and the sum is over the $M$ energy bins. If the coefficients $\alpha_j$ are chosen to be proportional to the average energies of the respective energy bins, this count signal $C_i$ will be proportional to the number of photocarriers created. In other embodiments, such as where the physical behavior to be corrected is related to changing circuit activity within the ASIC 130, the count signal may be proportional to the rate of the detection events being processed by the ASIC 130.

The count signal generated by the correction network 405 may be used to adjust the event thresholds set by the threshold generator 143. For example, a threshold correction algorithm may respond to changes in the count signal by generating threshold correction signals to adjust event thresholds for one or more energy bins in one or more pixels of the radiation detector. The threshold correction algorithm may be based on a training procedure described in further detail below. If the spectral shift is relatively uniform over all energy ranges, then the same threshold correction may be applied to each bin. On the other hand, if the spectral shift is significantly dependent on energy, different threshold corrections for each bin may be utilized. In some cases, the stability behavior of the pixels in response to an increasing flux may not be the same as for a decreasing flux. This may be because the time constants for the filling of carrier traps in the sensor material are not generally the same as the time constants for the emptying of the carrier traps. It may therefore be advantageous to incorporate nonlinear functions in the correction network 405.

The detector structure comprises a threshold adjustment compensation circuit 401 as further defined in the claims which may be implemented in the detector ASIC 130, for example, at the pixel level using individual correction of each pixel based on its own count history. In other embodiments, all or a portion of the threshold adjustment compensation circuit 401 may be implemented using at least one additional processor 140, such as a field programmable gate array (FPGA), as described above with reference to FIG. 2A. The additional processor 140 may process and aggregate all data for one or more ASICs 130.

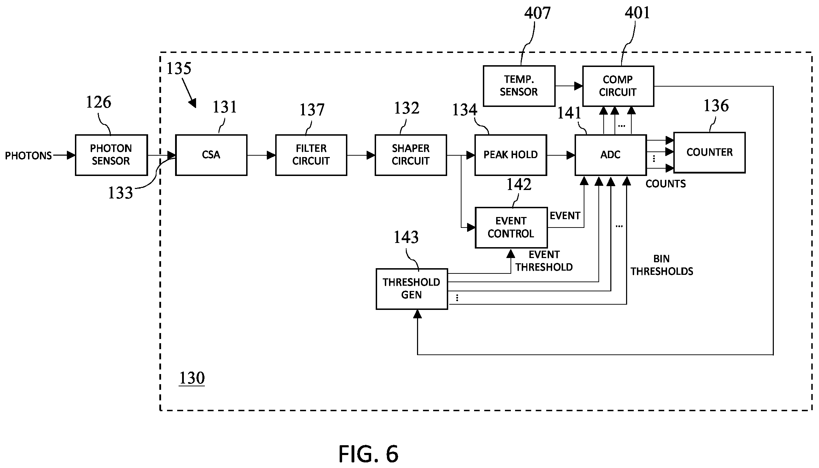

FIG. 6 is a block diagram schematically illustrating a signal chain architecture for one channel of a spectral photon counting (SPC) radiation detector including a threshold adjustment compensation circuit 401 and a temperature sensor 407 according to various embodiments of the present disclosure. As discussed above, in some cases the physical behavior of the radiation sensor 121 and/or the ASIC 130 may have a significant temperature dependence. A temperature sensor 407 may be provided in one or more locations in the detector to detect the temperature of the radiation sensor 121 and/or the ASIC 130. The detected temperature(s) may be provided to the threshold adjustment compensation circuit 401 which may generate threshold correction signals to adjust the event thresholds set by the threshold generator 143 based on both count data and temperature data. Although FIG. 6 illustrates a signal chain architecture similar to the signal chain architecture described above with reference to FIG. 4A, it will be understood that a temperature sensor 407 may also be used to provide temperature data to the threshold adjustment compensation circuit 401 in a switched-analog design architecture as described with reference to FIG. 4B.

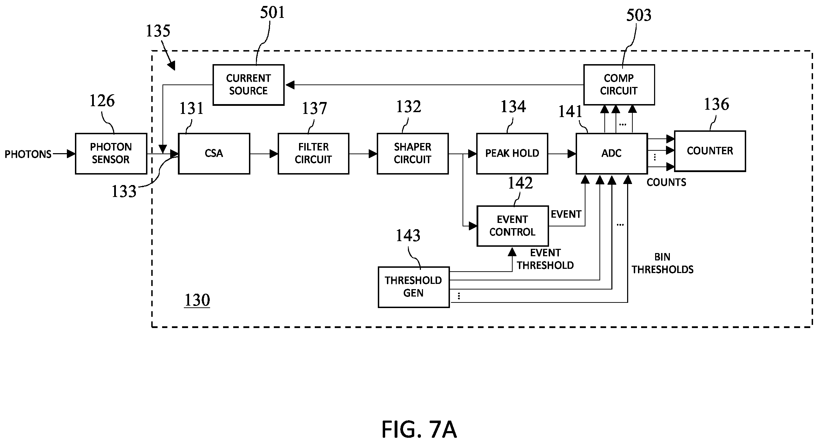

FIG. 7A is a block diagram schematically illustrating a signal chain architecture for one channel of a spectral photon counting (SPC) radiation detector including a current source 501 and a current adjustment compensation circuit 503 according to various embodiments of the present disclosure. Referring to FIG. 7A, the current source 501 may be coupled to the input of the amplifier 131 (e.g., CSA) of the signal processing channel 135. The current adjustment compensation circuit 503 may issue control signals to the current source 501 to cause the current source 501 to selectively apply a compensation current to the input of the amplifier 131. The compensation current applied to the input of the amplifier 131 may be configured to compensate for changes in the baseline current from the radiation sensor 121. For example, in instances in which there is an increase in the baseline DC current component (i.e., a "dark current" or "leakage current") in the output from the radiation sensor 121 that may contribute to a baseline shift to higher energies in the detector electronics, the current adjustment compensation circuit 503 may control the current source 501 to apply a compensating current to the input of the amplifier 131 having the same or similar magnitude as, and the opposite polarity to, the baseline current from the radiation sensor 121. This may partially or fully cancel out the baseline current without affecting the event detection signals.

In existing SPC detectors, it may be difficult if not impossible to accurately determine changes or shifts in the baseline current of the radiation sensor 121 under moderate or high flux conditions. This may be due to an increase in "pileup" effects, in which multiple photon interaction events may occur closely in space and time such that multiple pulses induced on the detector readout electronics temporally overlap with one another. In such situations, it may be difficult to isolate the baseline current from the overlapping photon detection signals. In addition, sudden increases in pileup events may be mistaken for an increase in the baseline current that has not actually occurred.

In various embodiments, the current adjustment compensation circuit 503 may be similar to the threshold adjustment compensation circuit 401 described above with reference to FIG. 5. In particular, photon count data for one or more pixels over each of the energy bins may be provided to the current adjustment compensation circuit 503 from the ADC 141 and/or the counter/accumulator 136. The count data may be processed (e.g., filtered and summed) as described above to provide a count signal that may be related to the incident flux. In some embodiments, the count signal (e.g., $C_i$) may be proportional to the number of photocarriers created within the sensor material. The count signal generated by the current adjustment compensation circuit 503 may be used to generate control signals configured to cause the current source 501 to apply a particular compensation current to the input of one or more signal processing channels 135 of the ASIC 130. For example, a current compensation algorithm may respond to changes in the count signal by generating control signals configured to cause the current source 501 apply a particular compensation current to the input of one or more signal processing channels 135 of the ASIC 130. The current compensation algorithm may be based on a training procedure described in further detail below.

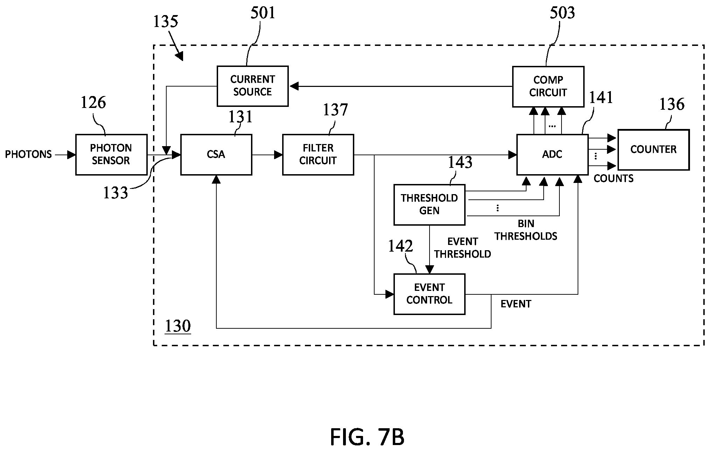

FIG. 7B is a block diagram schematically illustrating a signal chain architecture for one channel of a spectral photon counting (SPC) radiation detector including a current source 501 and a current adjustment compensation circuit 503 according to according to another embodiment of the present disclosure. The signal chain architecture shown in FIG. 7B may have a switched-analog design as described above with reference to FIG. 2C. As in the embodiment of FIG. 7A, the current adjustment compensation circuit 503 may receive photon count data from the ADC 141 and/or from the counter/accumulator 136, and the current adjustment compensation circuit 401 may control the current source 501 to selectively apply a compensation current to the signal processing channel 135 for the pixel.

A current adjustment compensation circuit 503 and a current source 501 as shown in FIGS. 7A and 7B may be advantageous for detector structures that exhibit a significant change in leakage current with changes in the incident photon flux. A current compensation scheme in accordance with the various embodiments may provide a compensating action at the physical effect causing the spectral shift, and may thereby achieve improved cancellation with a relatively straightforward circuit design. The current adjustment compensation circuit 503 may be trained for various flux patterns and spectra and may be effective during moderate and high flux operation.

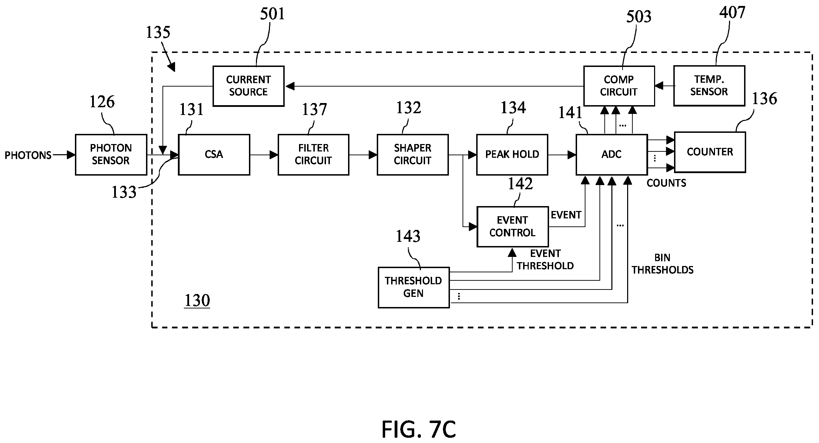

FIG. 7C is a block diagram schematically illustrating a signal chain architecture for one channel of a spectral photon counting (SPC) radiation detector including a current adjustment compensation circuit 503, a current source 501 and a temperature sensor 407 according to various embodiments of the present disclosure. A temperature sensor 407 as described above with reference to FIG. 6 may be provided in one or more locations in the detector to detect the temperature of the radiation sensor 121 and/or the ASIC 130. The detected temperature(s) may be provided to the current adjustment compensation circuit 503 which may generate control signals to control the compensation current applied by the current source 501 based on both count data and temperature data. Although FIG. 7C illustrates a signal chain architecture similar to the signal chain architecture described above with reference to FIG. 7A, it will be understood that a temperature sensor 407 may also be used to provide temperature data to the current adjustment compensation circuit 503 in a switched-analog design architecture as described with reference to FIG. 7B.

In some embodiments, an ASIC 130 of an SPC radiation detector may include both a threshold adjustment compensation circuit 401 as described in FIGS. 4A-6 and a current adjustment compensation circuit 503 as described in FIGS. 7A-7C.

As described above, the various embodiments are based on the recognition that the response of an SPC detector is a predictable function of the history of the photon flux incident on the detector. Accordingly, the SPC detector may be subjected to a series of training situations in which the flux may be varied in a variety of ways that are likely to be encountered under normal operating conditions. For example, the flux may be varied from a low or zero value to a high flux over a short period of time. Alternatively, the flux may change from a high to a moderate or to a low flux. This may be done using a series of filters that may be configured to vary the X-ray spectrum. In each case, the applied flux stimulus applied during each training situation may be known. Accordingly, photon counting results during each training session may be compared to provide stability metrics on varying time scales.

Since an "ideal" detector would have perfect stability performance (i.e., the stability metrics would all be zero), the stability metrics obtained from the training sessions may be provided as inputs to a machine learning or similar algorithm, which may be set up to adjust the various compensation parameters (e.g., threshold adjustments, current compensation) to drive the various stability values to approach the stability behavior of an "ideal" detector. After the algorithm has been sufficiently trained under a variety of variable flux conditions, it may be implemented as a feedback control algorithm in an above-described compensation circuit 401, 503 to adjust the various compensation parameters (e.g., threshold adjustments, current compensation) during operation of the SPC detector.

In some embodiments, this training process may not need to be performed for every pixel of the detector, or for every detector. In some cases, it may be advantageous to separately train different pixels of a detector that tend to exhibit different detector responses, such as performing separate trainings for edge pixels and interior pixels of the detectors. In the case of significant process changes in the manufacture, design and/or operation of the radiation sensors 121 and/or the ASICs 130, a retraining process may be performed.

In some embodiments, the training process may be performed over a range of temperatures. In cases in which it is determined that the detector response to changing flux conditions is also significantly temperature dependent, the algorithm may additionally be trained to adjust the various compensation parameters (e.g., threshold adjustments, current compensation) under varying temperature conditions. One or more temperature sensor signals may be included in the compensation circuit.

In some embodiments, the algorithm may be configured to adjust the filter constants and/or the time constants of the above-described filters 403 in the compensation circuit.

In some embodiments, a metric representing feedback stability may be developed for each data acquisition in the training procedure. The training algorithm may be configured to ensure that the control loop stability is adequately stable.

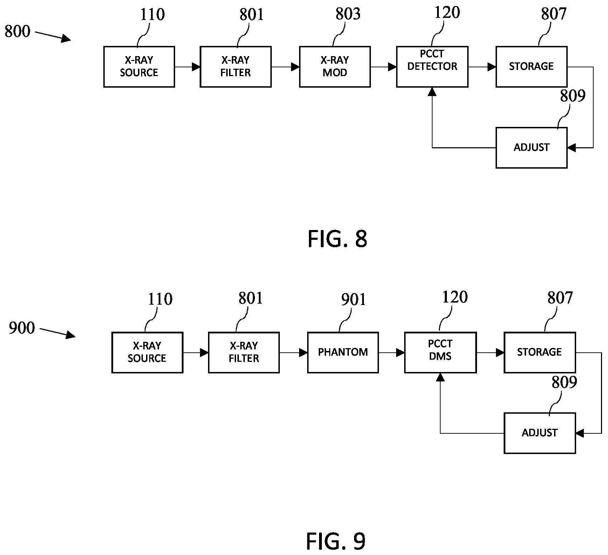

In some embodiments, the training procedure may be performed in the factory or in a similar controlled, factory-like setting where the X-ray stimulus may be readily controlled in a prescribed manner. FIG. 8 is a block diagram schematically illustrating an apparatus 800 that may be used for training of a feedback control algorithm to be implemented in a compensation circuit 401, 503 for an SPC detector according to various embodiments of the present disclosure. Referring to FIG. 8, the apparatus 800 may include an X-ray source 110 and an SPC radiation detector 120, such as a PCCT detector. A filter 801 may be provided in the beam path between the X-ray source 110 and the SPC radiation detector 120. The filter 801 may be configured to modify the energy spectrum of the output X-ray beam in a known manner. A modulator 803 may be operatively coupled to the X-ray source 110. The modulator 803 may be an X-ray shutter, capable of changing the attenuation in the X-ray path from a very high value, sufficient to ensure only background counts are registered in the detector 120, to a very low attenuation, preferably close to or equal to zero, where the shutter is wide open. Such a shutter may cause a rapid change in the incident photon flux, such as on the time scale of the response of the sensor material of the detector 120, e.g., much faster than 0.1 s, preferably on a time scale of 3 ms from fully closed to fully open. Such a shutter may be electromechanical or pneumatic. The counts in each bin from each pixel may be stored in a suitable storage medium 807 (e.g., a memory), and may be analyzed by a compensation adjustment unit 809, which may involve a machine learning algorithm as described above. In some embodiments, the time at which the change in flux occurs may be determined by electrical synchronization of a shutter driver with the acquisition system. In other embodiments, the time at which the change in flux occurs may be determined by analysis of the count waveforms, and by finding the point at which the counts increase suddenly from near zero in the dark to high values when the shutter is open. The compensation values in the feedback control algorithm may initially be set to zero or to other suitable values. Then a series of measurements can be made, and the compensation values may be adjusted. The process may be done in just one step, or in a series of steps, which may be seen as an iterative adjustment of the compensation values. It is likely that some compromise between performance in different use cases, e.g., flux levels or spectra may be required. In such a case, a desirability function may be used to weight the relative importance of performance in the different situations, and this can be used in the training process.

The use of factory training is convenient and preferable if it is effective. However, should there be any evolution in the detector response, such a procedure may not be adequate. Furthermore, it may be advantageous to train the SPC detectors in the actual environment in which imaging is performed, i.e., when the detectors are arranged in a CT gantry in a detector module system (DMS). In this way, any detector evolution can be compensated by recalibration with training. In addition, training inputs relating to system image quality (IQ) performance may also be incorporated.

FIG. 9 is a block diagram schematically illustrating an imaging system 900 that may be used for training of a feedback control algorithm to be implemented in a compensation circuit 401, 503 for an SPC detector according to various embodiments of the present disclosure. In such an imaging system 900, a phantom 901, i.e., an arrangement of X-ray absorbing material(s), may be disposed in the space between the X-ray source 110 and the SPC detector 120. In some embodiments, the imaging system 900 may be a PCCT system, and the phantom 901 may be located within the scanning volume of the PCCT system. In some embodiments, the phantom 901 may optionally be placed in the scanner after an air scan has been performed. In some embodiments 901, the phantom 901 may have a length along the z-axis direction (i.e., along the central axis of the bore of X-ray gantry) that is short enough that phantom 901 may be translated into the scanning volume and/or the X-ray gantry may be translated with respect to the phantom 901 to bring the phantom 901 into the scanning volume. When in use, the phantom 901 may affect the transmission of the X-ray beam between the X-ray source 110 and the SPC detector 120 (e.g., a PCCT DMS). In some embodiments, the X-ray beam may also be filtered using a suitable filter 801 as described above with reference to FIG. 8. During the training sequence, the CT gantry may rotate at a known speed, and the X-ray source 110 may be turned on and off at suitable times, to achieve the varying flux conditions at the SPC detector 120. The training procedure may proceed in a similar way to that described above for factory training. In the imaging system 901 of FIG. 9, the photon counts stored in storage medium 807 may be analyzed to develop stability metrics in view of the structure of the phantom 901. This may include determination of the timing of the counting signals with respect to the attenuation function of the phantom, either by electrical synchronization, or preferably, by analysis of the counting waveforms. The training may occur in the projection domain (i.e., prior to tomographic reconstruction of the raw image data), and/or within in the image domain (i.e., on the reconstructed CT image).

Imaging phantoms 901 are typically designed to provide imaging challenges typical of medical applications of CT imaging, e.g., with structures representative of various anatomic components and/or pathologies. Such phantoms may prove usable for the present in-gantry detector training function. However, it may be preferable to construct specialized phantoms for the training process. Such specialized phantoms may be disposed in a linear way along the Z axis of the scanner before or after medical imaging phantoms, i.e., multi-function phantoms may be constructed. The phantoms may be designed for use with Z translation, or not.

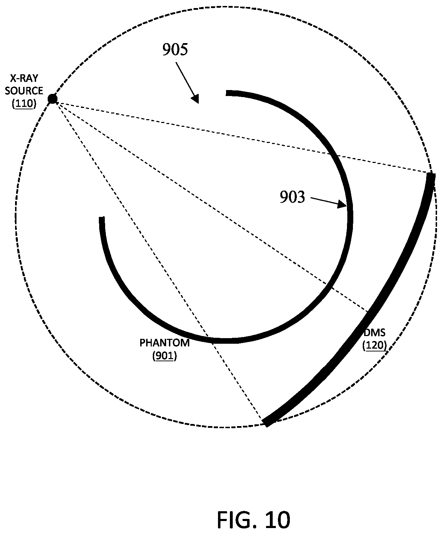

An example phantom 901 for stepwise flux modulation is shown in FIG. 10. The phantom 901 in this embodiment includes a blocking portion 903 shown in black, which in this example, fills ¾ of one rotation, i.e., 270 degrees, and provides a complete or near-complete attenuation of the X-ray beam. The phantom 901 may be composed of a band of tungsten or other highly absorbing material. The phantom 901 additionally includes an open region 905 that allows full or nearly-full transmission of the X-ray beam. This structure allows the X-ray beam to be turned on while the beam is blocked, and the gantry rotation will result in a sudden increase in flux at the point where the X-ray path is unblocked. The time at which the flux increases will vary from one pixel to another according to the geometry shown in FIG. 10. The X-ray source 110 may be turned off after the beam is blocked by further rotation, or it may be left on to allow a sequence of low and high fluxes to be achieved. Other arrangements may have a larger or smaller coverage of the phantom, i.e., coverage higher or lower than 270 degrees.

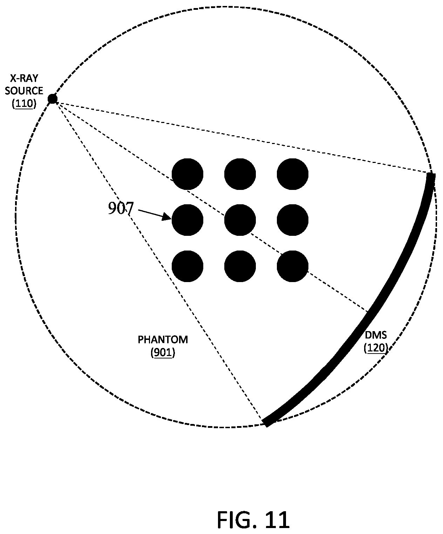

Other phantoms may be constructed from arrays of absorbing rods 907 of various materials, e.g., metals. Such an arrangement is shown in FIG. 11. While the phantom 901 in FIG. 11 does not achieve full zeroing of the flux, it may be combined with switch-on of the X-ray source 110 to provide a sudden change from zero to a high flux, where that high flux is then modulated over time. A phantom 901 as shown in FIG. 11 may more closely simulate the types of changing flux situations that the SPC detector 120 may experience in a medical imaging situation. In the embodiment shown in FIG. 11, it may be preferable to use a calculation of the X-ray absorption of the phantom 901 as a function of gantry rotation to identify the times at which the flux achieves identical values, which can then be used to define generalized stability metrics for training. The phantom 901 may include a plurality of metal rods 907 in a uniform matrix of absorbing material that is similar in absorption to water, etc. The phantom 901 may have, for example, a cylindrical shape. Other suitable shapes for the phantom 901 may also be utilized.

The devices (e.g., ASICs and/or detector structures including the ASICs) may include the features of various embodiments individually or any combination of two or more of such features. The devices of the embodiments of the present disclosure can be employed in various radiation detection systems including computed tomography (CT) imaging systems. Any direct conversion radiation sensors may be employed such as radiation sensors employing Si, Ge, GaAs, CdTe, CdZnTe, and/or other similar semiconductor materials.

The radiation detectors of the present embodiments may be used for medical imaging, such as in Low-Flux applications in Nuclear Medicine (NM), whether by Single Photon Emission Computed Tomography (SPECT) or by Positron Emission Tomography (PET), or as radiation detectors in High-Flux applications as in X-ray Computed Tomography (CT) for medical applications, and for non-medical imaging applications, such as in baggage security scanning and industrial inspection applications.

**Claims**

1.  A detector structure, comprising:

    at least one radiation sensor (121) comprising an array of pixel detectors;
    an application specific integrated circuit, ASIC (130), comprising a plurality of signal processing channel circuits ($135_1$, $135_2$, ... $135_n$) electrically coupled to respective pixel detectors of the array of pixel detectors, each signal processing channel circuit configured to generate photon count data for multiple energy bins for a respective pixel detector of the array of pixel detectors; and
    at least one compensation circuit (401) that receives photon count data for multiple energy bins from one or more signal processing channel circuits and adjusts a response characteristic of at least one signal processing channel circuit of the ASIC based on the received photon count data,
    wherein the at least one compensation circuit comprises a threshold adjustment compensation circuit (143) that is configured to adjust one or more threshold levels of the energy bins in at least one signal processing channel circuit of the ASIC based on the received photon count data,
    wherein each of the signal processing channel circuits comprises an amplifier (131) coupled to a respective pixel detector of the array of pixel detectors that produces an analog detection signal related to energy of photons detected by the pixel detector and an analog-to-digital conversion circuit that converts the analog detection signal to digital photon count data for each of the multiple energy bins, said detector structure being **characterized in that**
the at least one compensation circuit further comprises a plurality of filters (403) that filter the digital photon count data for each of the energy bins and a weighted summing network that combines the outputs from the plurality of filters to generate a count signal that is related to incident photon flux on one or more pixel detectors;
the count signal is proportional to photocarrier generation within the one or more pixel detectors or to a rate of detection signals processed by the ASIC;
the count signal is generated using digital photon count data from multiple signal processing channels of the ASIC;
time constants of the filters are set to correspond to physical time constants for changes in electric field that occur in the radiation sensor in response to changes in the incident photon flux;
the at least one compensation circuit implements a feedback control algorithm that adjusts the response characteristic of the at least one signal processing channel circuit of the ASIC based on changes in the count signal; and
the feedback control algorithm is trained using a training sequence that includes exposing the detector structure to changing incident photon flux conditions in a controlled environment.

2. The detector structure of claim 1, wherein the compensation circuit is located in the ASIC or at least partially on a separate processing device from the ASIC.

3. The detector structure of claim 1, wherein the threshold adjustment compensation circuit applies a same adjustment to each of the threshold levels of the energy bins in the at least one signal processing channel circuit of the ASIC based on the received photon count data.

4. The detector structure of claim 1, wherein the threshold adjustment compensation circuit applies individual adjustments to one or more threshold levels of the energy bins in the at least one signal processing channel circuit of the ASIC based on the received photon count data.

5. The detector structure of claim 1, wherein the threshold adjustment compensation circuit that is configured to adjust one or more threshold levels of the energy bins in at least one signal processing channel circuit of the ASIC during an imaging operation.

6. The detector structure of claim 1, wherein:

the ASIC further comprises a current source coupled to an input node of at least one signal processing channel circuit; and
the at least one compensation circuit comprises a current adjustment compensation circuit that is configured to control the current source to selectively apply a compensation current to the input node of the at least one signal processing channel circuit based on the received photon count data.

7. The detector structure of claim 1, further comprising a temperature sensor configured to monitor a temperature of the detector structure and to provide temperature data to the at least one compensation circuit, wherein the response characteristic of the at least one signal processing channel circuit of the ASIC is adjusted based on the received photon count data and the temperature data.

8. An X-ray imaging system, comprising:

a radiation source configured to emit X-rays; and
a detector array including a plurality of detector structures of claim 1 that form a continuous detector surface and that are configured to receive the X-rays from the radiation source through an intervening space configured to contain an object therein,
wherein the X-ray imaging system comprises a photon-counting computerized tomography (PCCT) imaging system comprising an image reconstruction system including a computer configured to run an automated image reconstruction algorithm using the photon count data generated by the detector structures of the detector array.

9. A method of using the detector structure of claim 1, comprising:

providing radiation to the at least one radiation sensor comprising the array of pixel detectors;
generating photon count data for multiple energy bins for the respective pixel detector of the array of pixel detectors using the application specific integrated circuit (ASIC) including the plurality of signal processing channel circuits electrically coupled to the respective pixel detectors of the array of pixel detectors; and
adjusting the response characteristic of the at least one signal processing channel circuit of the ASIC based on the photon count data for the multiple energy bins provided from the one or more signal processing channel circuits.

## Patentansprüche

1. Detektorstruktur, umfassend:

mindestens einen Strahlungssensor (121) umfassend ein Array von Pixeldetektoren; einen anwendungsspezifischen integrierten Schaltkreis, ASIC (130), der eine Vielzahl von Signalverarbeitungkanalschaltungen ($135_1$, $135_2$, ... $135_n$) umfasst, die elektrisch mit den jeweiligen Pixeldetektoren des Arrays von Pixeldetektoren gekoppelt sind, wobei jede Signalverarbeitungkanalschaltung so konfiguriert ist, dass sie Photonenzähldaten für mehrere Energiebehälter für einen jeweiligen Pixeldetektor des Arrays von Pixeldetektoren erzeugt; und mindestens eine Kompensationsschaltung (401), die Photonenzähldaten für mehrere Energiebehälter von einer oder mehreren Signalverarbeitungkanalschaltungen empfängt und eine Ansprechcharakteristik mindestens einer Signalverarbeitungkanalschaltung der ASIC auf der Grundlage der empfangenen Photonenzähldaten anpasst, wobei die mindestens eine Kompensationsschaltung eine Schwellenwertanpassungskompensationsschaltung (143) umfasst, die so konfiguriert ist, dass sie einen oder mehrere Schwellenwerte der Energiebehälter in mindestens einer Signalverarbeitungsschaltung des ASIC auf der Grundlage der empfangenen Photonenzählungsdaten anpasst, wobei jede der Signalverarbeitungskanalkreise einen Verstärker (131) umfasst, der mit einem entsprechenden Pixeldetektor des Arrays von Pixeldetektoren gekoppelt ist, der ein analoges Detektionssignal erzeugt, das sich auf die Energie der vom Pixeldetektor detektierten Photonen bezieht, und eine Analog-Digital-Wandlerschaltung, die das analoge Detektionssignal für jeden der mehreren Energiebehälter in digitale Photonenzähldaten umwandelt, wobei die Detektorstruktur **dadurch gekennzeichnet ist, dass** mindestens eine Kompensationsschaltung weiterhin eine Vielzahl von Filtern (403) umfasst, die die digitalen Photonenzähldaten für jeden der Energiebehälter filtern, sowie ein gewichtetes Summiernetzwerk, das die Ausgänge der Vielzahl von Filtern kombiniert, um ein Zählsignal zu erzeugen, das mit dem einfallenden Photonenfluss auf einem oder mehreren Pixeldetektoren in Beziehung steht; das Zählsignal proportional zur Photonenträgererzeugung innerhalb des einen oder der mehreren Pixeldetektoren oder zu einer Rate von Detektionssignalen ist, die von dem ASIC verarbeitet werden; das Zählsignal unter Verwendung digitaler Photonenzähldaten aus mehreren Signalverarbeitungskanälen des ASIC erzeugt wird; die Zeitkonstanten der Filter so eingestellt werden, dass sie den physikalischen Zeitkonstanten für Änderungen des elektrischen Feldes entsprechen, die im Strahlungssensor als Reaktion auf Änderungen des einfallenden Photonenflusses auftreten; die mindestens eine Kompensationsschaltung einen Rückkopplungsregelalgorithmus implementiert, der die Ansprechcharakteristik der mindestens einen Signalverarbeitungsschaltung des ASIC auf der Grundlage von Änderungen des Zählsignals anpasst; und der Rückkopplungsregelungsalgorithmus unter Verwendung einer Trainingssequenz trainiert wird, die das Aussetzen der Detektorstruktur wechselnden einfallenden Photonenflussbedingungen in einer kontrollierten Umgebung umfasst.

2. Detektorstruktur nach Anspruch 1, wobei die Kompensationsschaltung in dem ASIC oder zumindest teilweise auf einer von dem ASIC getrennten Verarbeitungseinrichtung angeordnet ist.

3. Detektorstruktur nach Anspruch 1, wobei die Schwellenwertanpassungskompensationsschaltung eine gleiche Anpassung an jeden der Schwellenwerte der Energiebehälter in der mindestens einen Signalverarbeitungsschaltung des ASIC auf der Grundlage der empfangenen Photonenzähldaten vornimmt.

4. Detektorstruktur nach Anspruch 1, wobei die Schwellenwertanpassungskompensationsschaltung individuelle Anpassungen an einem oder mehreren Schwellenwerten der Energiebehälter in der mindestens einen Signalverarbeitungkanalschaltung des ASIC auf der Grundlage der empfangenen Photonenzähldaten vornimmt.

5. Detektorstruktur nach Anspruch 1, wobei die Schwellenwertanpassungskompensationsschaltung so konfiguriert ist, dass sie einen oder mehrere Schwellenwerte der Energiebehälter in mindestens einer Signalverarbeitungsschaltung des ASIC während eines Bildgebungsvorgangs anpasst.

6. Detektorstruktur nach Anspruch 1, wobei:

der ASIC weiterhin eine Stromquelle umfasst, die mit einem Eingangsknoten mindestens einer Signalverarbeitungkanalschaltung gekoppelt ist; und

die mindestens eine Kompensationsschaltung eine Stromanpassungskompensationsschaltung umfasst, die so konfiguriert ist, dass sie die Stromquelle steuert, um selektiv einen Kompensationsstrom an den Eingangsknoten der mindestens einen Signalverarbeitungkanalschaltung auf der Grundlage der empfangenen Photonenzähldaten anzulegen.

7. Detektorstruktur nach Anspruch 1, weiterhin umfassend einen Temperatursensor, der so konfiguriert ist, dass er eine Temperatur der Detektorstruktur überwacht und Temperaturdaten an die mindestens eine Kompensationsschaltung liefert, wobei die Ansprechcharakteristik der mindestens einen Signalverarbeitungsschaltung des ASIC auf der Grundlage der empfangenen Photonenzähldaten und der Temperaturdaten angepasst wird.

8. Röntgenbildgebungssystem, umfassend:

eine Strahlungsquelle, die so konfiguriert ist, dass sie Röntgenstrahlen emittiert; und ein Detektorarray mit einer Vielzahl von Detektorstrukturen gemäß Anspruch 1, die eine durchgehende Detektoroberfläche bilden und so konfiguriert sind, dass sie die Röntgenstrahlen von der Strahlungsquelle durch einen dazwischen liegenden Raum empfangen, der so konfiguriert ist, dass er ein Objekt enthält, wobei das Röntgenbildgebungssystem ein Photonenzähl-Computertomographie-Bildgebungssystem (PCCT) umfasst, das ein Bildrekonstruktionssystem mit einem Computer umfasst, der so konfiguriert ist, dass er einen automatisierten Bildrekonstruktionsalgorithmus unter Verwendung der von den Detektorstrukturen der Detektorarray erzeugten Photonenzähldaten ausführt.

9. Verfahren zur Verwendung der Detektorstruktur nach Anspruch 1, umfassend:

Bereitstellen von Strahlung für den mindestens einen Strahlungssensor, der das Array von Pixeldetektoren umfasst;
Erzeugen von Photonenzähldaten für mehrere Energiebehälter für den jeweiligen Pixeldetektor des Arrays von Pixeldetektoren unter Verwendung der anwendungsspezifischen integrierten Schaltung (ASIC), die die mehreren Signalverarbeitungskanalschaltungen umfasst, die elektrisch mit den jeweiligen Pixeldetektoren des Arrays von Pixeldetektoren gekoppelt sind; und

Anpassen der Ansprechcharakteristik der mindestens einen Signalverarbeitungkanalschaltung der ASIC auf der Grundlage der Photonenzähldaten für die mehreren Energiebehälter, die von der einen oder den mehreren Signalverarbeitungkanalschaltungen bereitgestellt werden.

## Revendications

1. Structure de détecteur comprenant :

au moins un capteur de rayonnement (121) comprenant un réseau de détecteurs à pixels ;
un circuit intégré spécifique à une application, ASIC (130), comprenant une pluralité de circuits de canaux de traitement de signal ($135_1$, $135_2$, ... $135_n$) couplés électriquement aux détecteurs à pixels respectifs du réseau de détecteurs à pixels, chaque circuit de canaux de traitement de signal étant conçu pour générer des données de comptage de photons pour de multiples compartiments d'énergie pour un détecteur à pixels respectif du réseau de détecteurs à pixels ; et
au moins un circuit de compensation (401) qui reçoit des données de comptage de photons pour de multiples compartiments d'énergie provenant d'un ou plusieurs circuits de canaux de traitement de signal et qui règle une caractéristique de réponse d'au moins un circuit de canaux de traitement de signal de l'ASIC en fonction des données de comptage de photons reçues,
dans laquelle l'au moins un circuit de compensation comprend un circuit de compensation de réglage de seuil (143) qui est conçu pour régler un ou plusieurs niveaux de seuil des compartiments d'énergie dans au moins un circuit de canaux de traitement de signal de l'ASIC en fonction des données de comptage de photons reçues,
dans laquelle chacun des circuits de canaux de traitement de signal comprend un amplificateur (131) couplé à un détecteur à pixels respectif du réseau de détecteurs à pixels qui produit un signal de détection analogique relatif à l'énergie des photons détectés par le détecteur à pixels, et un circuit de conversion analogique-numérique qui convertit le signal de détection analogique en données numériques de comptage de photons pour chacun des multiples compartiments d'énergie, ladite structure de détecteur étant **caractérisée en ce que** l'au moins un circuit de compensation comprend en outre une pluralité de filtres (403) qui filtrent les données numériques de comptage de photons pour chacun des compartiments d'énergie, et un réseau de sommation pondérée qui combine les sorties provenant de la pluralité de filtres pour générer un signal de comptage qui est fonction du flux de photons incidents sur un ou plusieurs détecteurs à pixels ;

le signal de comptage est proportionnel à la génération de photoporteurs dans le ou les détecteurs à pixels ou à la fréquence des signaux de détection traités par l'ASIC ;

le signal de comptage est généré à l'aide de données numériques de comptage de photons provenant de multiples canaux de traitement de signal de l'ASIC ;

les constantes de temps des filtres sont définies pour correspondre aux constantes de temps physiques des variations du champ électrique qui se produisent dans le capteur de rayonnement en réponse aux variations du flux de photons incidents ;

l'au moins un circuit de compensation met en œuvre un algorithme de contrôle par rétroaction qui règle la caractéristique de réponse de l'au moins un circuit de canaux de traitement de signal de l'ASIC en fonction des variations du signal de comptage ; et

l'algorithme de contrôle par rétroaction est entraîné à l'aide d'une séquence d'entraînement qui inclut l'exposition de la structure de détecteur à des conditions de flux de photons incidents variables dans un environnement contrôlé.

2. Structure de détecteur selon la revendication 1, dans laquelle le circuit de compensation est situé dans l'ASIC ou au moins partiellement sur un dispositif de traitement distinct de l'ASIC.

3. Structure de détecteur selon la revendication 1, dans laquelle le circuit de compensation de réglage de seuil applique un même réglage à chacun des niveaux de seuil des compartiments d'énergie dans l'au moins un circuit de canaux de traitement de signal de l'ASIC, en fonction des données de comptage de photons reçues.

4. Structure de détecteur selon la revendication 1, dans laquelle le circuit de compensation de réglage de seuil applique des réglages individuels à un ou plusieurs niveaux de seuil des compartiments d'énergie dans l'au moins un circuit de canaux de traitement de signal de l'ASIC, en fonction des données de comptage de photons reçues.

5. Structure de détecteur selon la revendication 1, dans laquelle le circuit de compensation de réglage de seuil qui est conçu pour régler un ou plusieurs niveaux de seuil des compartiments d'énergie dans au moins un circuit de canaux de traitement de signal de l'ASIC pendant une opération d'imagerie.

6. Structure de détecteur selon la revendication 1, dans laquelle :

l'ASIC comprend en outre une source de courant couplée à un nœud d'entrée d'au moins un circuit de canaux de traitement de signal ; et

l'au moins un circuit de compensation comprend un circuit de compensation de réglage de courant qui est conçu pour commander la source de courant afin d'appliquer sélectivement un courant de compensation au nœud d'entrée de l'au moins un circuit de canaux de traitement de signal en fonction des données de comptage de photons reçues.

7. Structure de détecteur selon la revendication 1, comprenant en outre un capteur de température conçu pour surveiller une température de la structure de détecteur et pour fournir des données de température à l'au moins un circuit de compensation, dans laquelle la caractéristique de réponse de l'au moins un circuit de canaux de traitement de signal de l'ASIC est réglée en fonction des données de comptage de photons reçues et des données de température.

8. Système d'imagerie à rayons X, comprenant :

une source de rayonnement conçue pour émettre des rayons X ; et

un réseau de détecteurs incluant une pluralité de structures de détecteur selon la revendication 1 qui forment une surface de détection continue et qui sont conçues pour recevoir les rayons X de la source de rayonnement à travers un espace intermédiaire conçu pour contenir un objet,

dans lequel le système d'imagerie à rayons X comprend un système d'imagerie par tomodensitométrie à comptage de photons (PCCT) comprenant un système de reconstruction d'image incluant un ordinateur conçu pour exécuter un algorithme de reconstruction d'image automatisé utilisant les données de comptage de photons générées par les structures de détecteur du réseau de détecteurs.

9. Procédé d'utilisation de la structure de détecteur selon la revendication 1, comprenant :

la fourniture d'un rayonnement à l'au moins un capteur de rayonnement comprenant le réseau de détecteurs à pixels ;

la génération de données de comptage de photons pour de multiples compartiments d'énergie pour le détecteur à pixels respectif du réseau de détecteurs à pixels à l'aide du circuit intégré spécifique à une application (ASIC) incluant la pluralité de circuits de canaux de traitement de signal couplés électriquement aux détecteurs à pixels respectifs du réseau de détecteurs à pixels ; et

le réglage de la caractéristique de réponse de l'au moins un circuit de canaux de traitement de signal de l'ASIC en fonction des données de comptage de photons pour les multiples compartiments d'énergie fournies par le ou les circuits de canaux de traitement de signal.

FIG. 1A

FIG. 1B

FIG. 2A

EP 4 528 335 B1

FIG. 2B

FIG. 2C

FIG. 3

135

401

136 COUNTER

COMP CIRCUIT

141 ADC

COUNTS

134 PEAK HOLD

142 EVENT CONTROL

EVENT

EVENT THRESHOLD

143 THRESHOLD GEN

BIN THRESHOLDS

132 SHAPER CIRCUIT

137 FILTER CIRCUIT

131 CSA

130

126 PHOTON SENSOR

133

PHOTONS

FIG. 4A

FIG. 4B

FIG. 5

FIG. 6

FIG. 7A

FIG. 7B

FIG. 7C

800 →

| 110 | 801 | 803 | 120 | 807 |
|---|---|---|---|---|
| X-RAY SOURCE | X-RAY FILTER | X-RAY MOD | PCCT DETECTOR | STORAGE |

809

ADJUST

## FIG. 8

900 →

| 110 | 801 | 901 | 120 | 807 |
|---|---|---|---|---|
| X-RAY SOURCE | X-RAY FILTER | PHANTOM | PCCT DMS | STORAGE |

809

ADJUST

## FIG. 9

FIG. 10

X-RAY
SOURCE
(110)

907

PHANTOM
(901)

DMS
(120)

FIG. 11

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20160203620 A1
- US 20070098139 A1
- US 20210382188 A1
- US 20210186439 A1
- US 31898723